# EUROPEAN PATENT APPLICATION

(11) **EP 4 656 633 A1**
(43) Date of publication of application: **03.12.2025**
(21) Application number: 24747202.0
(22) Date of filing: 18.01.2024
(51) Int. Cl.: C07D 331/02, C08G 75/08, G02B 1/04

(54) **EPISULFIDE COMPOSITION, POLYMERIZABLE COMPOSITION, CURED PRODUCT, OPTICAL MATERIAL, AND LENS**

(30) Priority: 25.01.2023 JP 2023009655
(71) Applicant: Mitsui Chemicals, Inc., Tokyo 104-0028 (JP)
(72) Inventor: FURUYA, Masayuki, Omuta-shi, Fukuoka 836-8610 (JP)
(74) Representative: Mewburn Ellis LLP
(86) International application number: PCT/JP2024/001277
(87) International publication number: WO 2024/157873

(57) **Abstract**

An episulfide composition containing an compound A, wherein: the compound A comprises a disulfide bond, an episulfide ring 1 containing an asymmetric carbon C*¹, and an episulfide ring 2 containing an asymmetric carbon C*², wherein the compound A comprises: at least one of a compound A-SS in which both of the C*¹ and the C*² have an S configuration, or a compound A-RR in which both of the C*¹ and the C*² have an R configuration; and a compound A-RS in which one of the C*¹ and the C*² has an R configuration, and another has an S configuration; and wherein a ratio of a peak area RS of the compound A-RS to a total peak area T of the compound A-SS, the compound A-RR and the compound A-RS, as measured by HPLC, is 10 to 70 area %.

## Description

### Technical Field

The present disclosure relates to an episulfide composition, a polymerizable composition, a cured product, an optical material, and a lens.

### Background Art

Plastic lenses are lighter in weight, less susceptible to cracking and can be more easily colored as compared to inorganic lenses, and thus are becoming rapidly popular for use in optical materials such as eyeglass lenses and camera lenses.

Of these, optical materials made of resins using episulfide compounds as raw materials are widely used, because such optical materials have an excellent refractive index and the like (see Patent Document 1, for example).

Patent Document 1: Japanese Patent Application Laid-Open (JP-A) No. 2002-194083

### SUMMARY OF THE INVENTION

### Technical Problem

However, there is a case in which a cured product obtained using an episulfide compound and an optical material including the cured product are required to have a more reduced haze.

The present disclosure has been made in view of the actual situation as described above.

An object of the present disclosure is to provide an episulfide composition and a polymerizable composition capable of providing a cured product having a reduced haze, as well as a cured product, an optical material and a lens having a reduced haze.

### Solution to Problem

Means for solving the above-mentioned problem include the following embodiments.
<1> An episulfide composition comprising an optically active compound A,
   wherein:
   the optically active compound A comprises one disulfide bond and two episulfide rings, and
   one of the two episulfide rings is an episulfide ring 1 containing an asymmetric carbon atom C*¹, and another of the two episulfide rings is an episulfide ring 2 containing an asymmetric carbon atom C*²,
   wherein the optically active compound A comprises:
      at least one of an optically active compound A-SS in which both of the asymmetric carbon atom C*¹ and the asymmetric carbon atom C*² have an S configuration, or an optically active compound A-RR in which both of the asymmetric carbon atom C*¹ and the asymmetric carbon atom C*² have an R configuration; and
      an optically active compound A-RS in which one of the asymmetric carbon atom C*¹ and the asymmetric carbon atom C*² has an R configuration, and another of the asymmetric carbon atoms has an S configuration; and
   wherein a ratio of a peak area RS of the optically active compound A-RS to a total peak area T of the optically active compound A-SS, the optically active compound A-RR and the optically active compound A-RS, as measured by high performance liquid chromatography, is from 10% by area to 70% by area.
<2> The episulfide composition according to <1>, wherein the ratio of the peak area RS to the total peak area T is more than 50% by area and less than or equal to 70% by area.
<3> The episulfide composition according to <1>, wherein the ratio of the peak area RS to the total peak area T is from 10% by area to 50% by area.
<4> The episulfide composition according to any one of <1> to <3>, wherein the optically active compound A comprises a compound represented by the following Formula (A1): 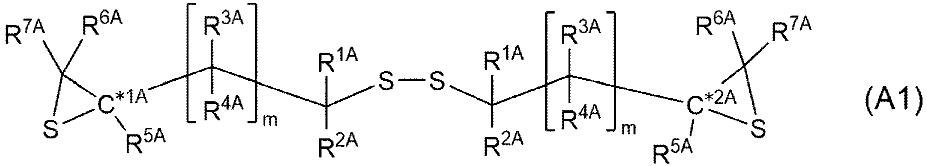 wherein, in Formula (A1), each of R^{1A} to R^{7A} independently represents a hydrogen atom or an optionally substituted monovalent hydrocarbon group; m represents an integer from 0 to 3; C*^{1A} represents the asymmetric carbon atom C*¹ in the compound represented by Formula (A1), and C*^{2A} represents the asymmetric carbon atom C*² in the compound represented by Formula (A1); and each of R^{1A} to R^{7A} and , which are present in pluralities, may be the same as, or different from, each other.
<5> The episulfide composition according to any one of <1> to <4>, wherein a content of the optically active compound A is 90% by mass or more with respect to a total amount of the episulfide composition.
<6> A polymerizable composition comprising the episulfide composition according to any one of <1> to <5>, and a polymerization catalyst.
<7> The polymerizable composition according to <6>, wherein the polymerizable composition is used in production of an optical material.
<8> A cured product of the polymerizable composition according to <6>.
<9> An optical material comprising a cured product of the polymerizable composition according to <6>.
<10> A lens comprising a cured product of the polymerizable composition according to <6>.

### Advantageous Effects of Invention

The present disclosure provides an episulfide composition and a polymerizable composition capable of providing a cured product having a reduced haze, as well as a cured product, an optical material and a lens having a reduced haze.

### DESCRIPTION OF EMBODIMENTS

In the present disclosure, any numerical range indicated using the expression "from * to" represents a range in which numerical values described before and after the "to" are included in the range as a lower limit value and an upper limit value.

In a case in which a plurality of substances corresponding to each component are present in a composition, in the present disclosure, the amount of each component in the composition refers to the total amount of the plurality of substances present in the composition, unless otherwise defined.

In numerical ranges described in stages, in the present disclosure, an upper limit value or a lower limit value described in one numerical range may be replaced with an upper limit value or a lower limit value of another numerical range in stages. Further, in a numerical range described in the present disclosure, the upper limit value or the lower limit value of the numerical range may be replaced with a value shown in Examples.

### [Episulfide Composition]

The episulfide composition according to the present disclosure is
an episulfide composition containing an optically active compound A,
wherein:
   the optically active compound A contains one disulfide bond and two episulfide rings, and
   one of the two episulfide rings is an episulfide ring 1 containing an asymmetric carbon atom C*¹, and the other one of the two episulfide rings is an episulfide ring 2 containing an asymmetric carbon atom C*²,
wherein the optically active compound A includes:
   at least one of an optically active compound A-SS in which both of the asymmetric carbon atom C*¹ and the asymmetric carbon atom C*² have an S configuration, or an optically active compound A-RR in which both of the asymmetric carbon atom C*¹ and the asymmetric carbon atom C*² have an R configuration; and
   an optically active compound A-RS in which one of the asymmetric carbon atom C*¹ and the asymmetric carbon atom C*² has an R configuration, and the other one of the asymmetric carbon atoms has an S configuration; and
wherein the ratio (hereinafter, also referred to as "peak area ratio [RS/T])" of the peak area RS of the optically active compound A-RS to the total peak area T of the optically active compound A-SS, the optically active compound A-RR and the optically active compound A-RS, as measured by high performance liquid chromatography, is from 10% by area to 80% by area.

The episulfide composition according to the present disclosure enables the obtaining of a cured product having a reduced haze.

The fact that the peak area ratio [RS/T] is 80% by area or less is thought to contribute to the effect of reducing the haze of the resulting cured product.

The fact that the peak area ratio [RS/T] is 10% by area or more is thought to contribute to the curability and the handleability of the episulfide composition.

### < Optically Active compound A >

The episulfide composition according to the present disclosure contains an optically active compound A.

The optically active compound A is a compound containing one disulfide bond and two episulfide rings.

In other words, the optically active compound A is a compound included in the concept of an episulfide compound (namely, a compound containing an episulfide ring).

One of the two episulfide rings in the optically active compound A is an episulfide ring 1 containing an asymmetric carbon atom C*¹, and the other one of the two episulfide rings is an episulfide ring 2 containing an asymmetric carbon atom C*².

The optically active compound A includes:
at least one of an optically active compound A-SS in which both of the asymmetric carbon atom C*¹ and the asymmetric carbon atom C*² have an S configuration, or an optically active compound A-RR in which both of the asymmetric carbon atom C*¹ and the asymmetric carbon atom C*² have an R configuration; and
an optically active compound A-RS in which one of the asymmetric carbon atom C*¹ and the asymmetric carbon atom C*² has an R configuration, and the other one of the asymmetric carbon atoms has an S configuration.

In the present disclosure, the "asymmetric carbon atom having an R configuration" refers to an asymmetric carbon atom whose steric configuration is an R configuration, and the "asymmetric carbon atom having an S configuration" refers to an asymmetric carbon atom whose steric configuration is an S configuration.

The optically active compound A-RS in which one of the asymmetric carbon atom C*¹ and the asymmetric carbon atom C*² has an R configuration, and the other one of the asymmetric carbon atoms has an S configuration, in the optically active compound A, is preferably a meso compound.

The fact that the optically active compound A-RS is a meso compound means that a compound in which the asymmetric carbon atom C*^{1A} has an S configuration and the asymmetric carbon atom C*^{2A} has an R configuration, and a compound in which the asymmetric carbon atom C*^{2A} has an R configuration and the asymmetric carbon atom C*^{2A} has an S configuration, are the same compound.

As described above, the optically active compound A includes at least one of the optically active compound A-SS or the optically active compound A-RR.

The optically active compound A more preferably includes both of the optically active compound A-SS and the optically active compound A-RR.

The optically active compound A preferably includes a compound represented by the following Formula (A1).

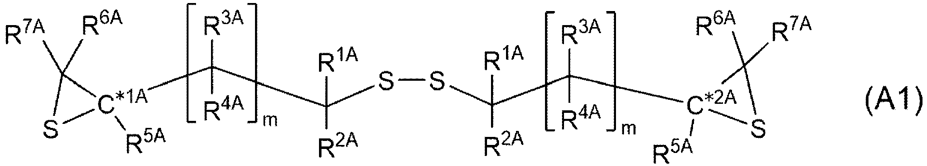

In Formula (A1), each of R^{1A} to R^{7A} independently represents a hydrogen atom or an optionally substituted monovalent hydrocarbon group; m represents an integer from 0 to 3; C*^{1A} represents the asymmetric carbon atom C*¹ in the compound represented by Formula (A1), and C*^{2A} represents the asymmetric carbon atom C*² in the compound represented by Formula (A1); and each of R^{1A} to R^{7A} and , which are present in pluralities, may be the same as, or different from, each other.

The optionally substituted monovalent hydrocarbon group represented by each of R^{1A} to R^{7A} in Formula (A1) may be, for example, a halogen atom, a thiol group (namely, a mercapto group), an alkoxy group, an alkylthio group, a hydroxyl group or the like.

The number of carbon atoms in the optionally substituted monovalent hydrocarbon group represented by each of R^{1A} to R^{7A} in Formula (A1) is preferably from 1 to 10, more preferably from 1 to 6, still more preferably from 1 to 3, yet still more preferably 1 or 2, and yet still more preferably 1, as the total number of carbon atoms including the number of carbon atoms in a substituent in a case in which the monovalent hydrocarbon group has a substituent.

The "optionally substituted monovalent hydrocarbon group" represented by each of R^{1A} to R^{7A} in Formula (A1) is preferably an alkyl group having from 1 to 6 carbon atoms (namely, an unsubstituted alkyl group), more preferably an alkyl group having from 1 to 3 carbon atoms, still more preferably a methyl group or an ethyl group, and yet still more preferably a methyl group.

In Formula (A1), each of R^{1A} to R^{7A} is particularly preferably a hydrogen atom.

In Formula (A1), m represents an integer from 0 to 3.
m is preferably an integer from 0 to 2, more preferably 0 or 1, and still more preferably 1.

In Formula (A1), C*^{1A} represents the asymmetric carbon atom C*¹ in the compound represented by Formula (A1), and C*^{2A} represents the asymmetric carbon atom C*² in the compound represented by Formula (A1).

Each of C*^{1A} and C*^{2A} may be an asymmetric carbon atom having an S configuration, or an asymmetric carbon atom having an R configuration.

The optically active compound A more preferably includes:
at least one of a compound (A1SS) which is a compound represented by Formula (A1) and in which compound both of C*^{1A} and C*^{2A} have an S configuration, or a compound (A1RR) which is a compound represented by Formula (A1) and in which compound both of C*^{1A} and C*^{2A} have an R configuration (more preferably both of these compounds); and
a compound (A1RS) which is a compound represented by Formula (A1) and in which compound one of the C*^{1A} and the C*^{2A} has an R configuration, and the other one thereof has an S configuration.

The content of the optically active compound A described above is preferably 80% by mass or more, and more preferably 90% by mass or more, with respect to the total amount of the episulfide composition.

Further, the total content of the optically active compound A-SS, the optically active compound A-RR and the optically active compound A-RS, is preferably 80% by mass or more, and more preferably 90% by mass or more, with respect to the total amount of the episulfide composition.

In addition, the total content of the compound (A1SS), the compound (A1RR) and the compound (A1RS), is preferably 80% by mass or more, and more preferably 90% by mass or more, with respect to the total amount of the episulfide composition.

### < Peak Area Ratio [RS/T] >

In the episulfide composition according to the present disclosure, the peak area ratio [RS/T] (namely, the ratio of the peak area RS of the optically active compound A-RS to the total peak area T of the optically active compound A-SS, the optically active compound A-RR and the optically active compound A-RS, as measured by high performance liquid chromatography) is from 10% by area to 70% by area.

Conditions for measuring the peak area ratio [RS/T] by high performance liquid chromatography (HPLC) are as will be shown in Examples to be described later.

When the peak area ratio [RS/T] is 70% by area or less, in the episulfide composition according to the present disclosure, the haze of the cured product of the episulfide composition according to the present disclosure is reduced.

When the peak area ratio [RS/T] is 10% by area or more, in the episulfide composition according to the present disclosure, the curability and the handleability of the episulfide composition according to the present disclosure are improved.

In the present disclosure, the concept of the cured product of the episulfide composition according to the present disclosure includes not only a cured product obtained by curing the episulfide composition according to the present disclosure alone, but also a cured product obtained by curing the polymerizable composition according to the present disclosure which will be described later (namely, a polymerizable composition containing the episulfide composition according to the present disclosure, and a polymerization catalyst).

Further, the concept of the curability of the episulfide composition according to the present disclosure, in the present disclosure, includes not only the curability of the episulfide composition according to the present disclosure alone, but also the curability of the polymerizable composition according to the present disclosure which will be described later (namely, a polymerizable composition containing the episulfide composition according to the present disclosure, and a polymerization catalyst).

As described above, the peak area ratio [RS/T] is required to be within a range of from 10% by area to 70% by area.

The peak area ratio [RS/T] is preferably from 20% by area to 70% by area, more preferably from 50% by area to 70% by area, and still more preferably more than 50% by area and less than or equal to 70% by area, from the viewpoint of further improving the maximum point stress of bending of the cured product of the episulfide composition.

Further, the peak area ratio [RS/T] may be from 10% by area to 50% by area.

### < Other Component(s) >

The episulfide composition according to the present disclosure may contain any other components other than the optically active compound A.

Examples of the other components include reaction solvents used for the synthesis of the optically active compound A, and reaction byproducts (impurities) produced by the synthesis.

### < Applications >

The applications of the episulfide composition according to the present disclosure are not particularly limited.

The episulfide composition according to the present disclosure is suitably used, for example, in production of an optical material, because the episulfide composition is capable of producing a cured product of the episulfide compound (specifically, the optically active compound A) which can have a high refractive index.

The optical material will be described later.

### [Polymerizable Composition]

The polymerizable composition according to the present disclosure contains the episulfide composition according to the present disclosure which has been described above, and a polymerization catalyst.

Since the polymerizable composition according to the present disclosure contains the above-described episulfide composition according to the present disclosure, the polymerizable composition has an excellent polymerizability.

### < Polymerization Catalyst >

The polymerizable composition according to the present disclosure contains a polymerization catalyst.

The polymerizable composition may contain one kind of polymerization catalyst, or two or more kinds of polymerization catalysts.

The polymerization catalyst may be, for example, a tertiary amine compound, a phosphine compound, a Lewis acid, a radical polymerization catalyst, a cation polymerization catalyst or the like.

For example, polymerization catalysts described in paragraphs from 0029 to 0033 in JP-A No. 2002-194083 can be referred to.

The polymerization catalyst is preferably a tertiary amine compound or a phosphine compound.

Examples of the tertiary amine compound include:
triethylamine,
tri-n-butylamine,
tri-n-hexylamine,
N,N-diisopropylethylamine,
triethylenediamine,
triphenylamine,
N,N-dimethylethanolamine,
N,N-diethylethanolamine,
N,N-dibutylethanolamine,
triethanolamine,
N-ethyldiethanolamine,
N,N-dimethylbenzylamine,
N,N-diethylbenzylamine,
tribenzylamine,
N-methyldibenzylamine,
N,N-dimethylcyclohexylamine,
N,N-dicyclohexylmethylamine,
N,N-diethylcyclohexylamine,
N,N-dicyclohexylethylamine,
N,N-dimethylbutylamine,
N,N-dicyclohexylbutylamine,
N-methylmorpholine,
N-isopropylmorpholine,
pyridine,
quinoline,
N,N-dimethylaniline,
N,N-diethylaniline,
α-picoline,
β-picoline,
γ-picoline,
2,2'-bipyridyl,
1,4-dimethylpiperazine,
tetramethylethylenediamine,
hexamethylenetetramine,
1,8-diazabicyclo(5,4,0)-7-undecene, and
2,4,6-tris (N,N-dimethylaminomethyl)phenol.

Examples of the phosphine compound include:
trimethylphosphine,
triethylphosphine,
tri-n-propylphosphine,
triisopropylphosphine,
tri-n-butylphosphine,
triphenylphosphine,
tribenzylphosphine,
1,2-bis(diphenylphosphino)ethane, and
1,2-bis(dimethylphosphino)ethane.

The content of the polymerization catalyst in the polymerizable composition according to the present disclosure is preferably from 0.01% by mass to 5% by mass, more preferably from 0.01% by mass to 2% by mass, and still more preferably from 0.03% by mass to 1% by mass, with respect to the total amount of the polymerizable composition.

### < Thiol Compound >

It is preferred that the polymerizable composition according to the present disclosure further contains a thiol compound.

In a case in which the polymerizable composition according to the present disclosure contains a thiol compound, it is possible to obtain a resin (namely, a cured product) by polymerizing an episulfide compound (A) and an episulfide compound (B) contained in episulfide composition, with the thiol compound. The resulting resin may have better resin properties, in this case.

In a case in which the polymerizable composition according to the present disclosure contains a thiol compound, the polymerizable composition may contain one kind of thiol compound, or two or more kinds of thiol compounds.

The thiol compound may be, for example, an aliphatic thiol, an aromatic thiol or the like.

The aliphatic thiol is preferably at least one selected from the group consisting of:
pentaerythritol tetrakis(2-mercaptothioglycolate),
pentaerythritol tetrakis(3-mercaptopropionate),
trimethylolpropane tris(2-mercaptothioglycolate),
trimethylolpropane tris(3-mercaptopropionate),
4-mercaptomethyl-1,8-dimercapto-3,6-dithiaoctane,
2,5-dimercaptomethyl-1,4-dithiane,
2,5-bis[(2-mercaptoethyl)thiomethyl]-1,4-dithiane,
1,3-cyclohexanedithiol,
1,4-cyclohexanedithiol,
bis(mercaptomethyl)sulfide,
bis(mercaptomethyl)disulfide,
bis(mercaptoethyl) sulfide,
bis(mercaptoethyl)disulfide,
4,8-dimercaptomethyl-1,11-dimercapto-3,6,9-trithiaundecane,
4,7-dimercaptomethyl-1,11-dimercapto-3,6,9-trithiaundecane,
5,7-dimercaptomethyl-1,11-dimercapto-3,6,9-trithiaundecane, and
tetrakis(mercaptomethylthio)propane.

However, the aliphatic thiol is not limited to the specific examples described above.

The aromatic thiol is preferably at least one selected from the group consisting of: benzylthiol,
xylylenedithiol,
1,3-bis(mercaptoethyl)benzene,
1,4-bis(mercaptoethyl)benzene
2,5-toluenedithiol,
3,4- toluenedithiol,
thiophenol,
1,2-dimercaptobenzene,
1,3-dimercaptobenzene,
1,4-dimercaptobenzene, and
1,2-bis(mercaptomethyl)benzene.

However, the aromatic thiol is not limited to the specific examples described above.

The thiol compound is preferably a compound (hereinafter, also referred to as "polythiol compound") having two or more thiol groups (namely, mercapto groups).

Further, in a case in which the polymerizable composition according to the present disclosure contains a thiol compound, the thiol compound preferably includes an aliphatic polythiol (namely, an aliphatic thiol which is a polythiol compound).

In this case, the proportion of the aliphatic polythiol in the total amount of the thiol compounds is preferably 50% by mass, more preferably 60% by mass or more, and still more preferably 80% by mass or more.

The upper limit of the proportion of the aliphatic thiol is not particularly limited. The proportion of the aliphatic thiol may be 100% by mass, less than 100% by mass, 99% by mass or less, or 95% by mass or less.

The thiol compound more preferably includes at least one (hereinafter, also referred to as "polythiol A") selected from the group consisting of:
4-mercaptomethyl-1,8-dimercapto-3,6-dithiaoctane,
4,8-dimercaptomethyl-1,11-dimercapto-3,6,9-trithiaundecane,
4,7-dimercaptomethyl-1,11-dimercapto-3,6,9-trithiaundecane, and
5,7-dimercaptomethyl-1,11-dimercapto-3,6,9-trithiaundecane.

In this case, the proportion of the polythiol A in the total amount of the thiol compounds is preferably 50% by mass, more preferably 60% by mass or more, and still more preferably 80% by mass or more.

The upper limit of the proportion of the polythiol A is not particularly limited. The proportion of the polythiol A may be 100% by mass, less than 100% by mass, 99% by mass or less, or 95% by mass or less.

The thiol compound more preferably includes at least one (hereinafter, also referred to as "polythiol A1") selected from the group consisting of:
4,8-dimercaptomethyl-1,11-dimercapto-3,6,9-trithiaundecane,
4,7-dimercaptomethyl-1,11-dimercapto-3,6,9-trithiaundecane, and
5,7-dimercaptomethyl-1,11-dimercapto-3,6,9-trithiaundecane.

In this case, the proportion of the polythiol A1 in the total amount of the thiol compounds is preferably 50% by mass, more preferably 60% by mass or more, and still more preferably 80% by mass or more.

The upper limit of the proportion of the polythiol A1 is not particularly limited. The proportion of the polythiol A1 may be 100% by mass, less than 100% by mass, 99% by mass or less, or 95% by mass or less.

In a case in which the episulfide composition according to the present disclosure contains a thiol compound, the content of the thiol compound is preferably from 1% by mass to 50% by mass, more preferably from 2% by mass to 30% by mass, still more preferably from 3% by mass to 20% by mass, and yet still more preferably from 5% by mass to 15% by mass, with respect to the total amount of a specific episulfide compound.

Thiol compounds described in paragraph 0039 in WO 2018/079829 may be referred to.

### < Other Component(s) >

The polymerizable composition according to the present disclosure may contain any other components other than the components described above.

Examples of the other components include isocyanate compounds, epoxy compounds, alcohol compounds (such as polyol compounds), mercapto organic acids, resins (such as acrylic resins and olefin resins), crosslinking agents, photostabilizers, ultraviolet absorbers, antioxidants, stain inhibitors, dyes, fillers, and internal mold release agents.

Any known components can be used as the other components.

For example, other components described in JP-A No. 2002-194083 can be referred to, as appropriate.

### < Applications >

The applications of the polymerizable composition according to the present disclosure are not particularly limited.

The polymerizable composition according to the present disclosure is suitably used, for example, in production of an optical material, because the polymerizable composition is capable of producing a cured product which can have a high refractive index. The optical material will be described later.

### [Cured Product]

The cured product according to the present disclosure is a cured product of the polymerizable composition according to the present disclosure.

The cured product according to the present disclosure is obtained, for example, by polymerizing monomers (such as the episulfide compound (A) and the episulfide compound (B); or alternatively, the episulfide compound (A), the episulfide compound (B) and the thiol compound; etc.) in the polymerizable composition according to the present disclosure, to cure the polymerizable composition according to the present disclosure. The cured product according to the present disclosure in this case includes a resin obtained by the polymerization of the monomers in the polymerizable composition according to the present disclosure.

The resulting resin has excellent resin properties.

The method of polymerizing the monomers in the polymerizable composition according to the present disclosure (namely, the method of producing the cured product according to the present disclosure) is not particularly limited.

Examples of the method of polymerizing the monomers in the polymerizable composition according to the present disclosure include cast polymerization.

In the cast polymerization, the polymerizable composition according to the present disclosure is first injected between a pair of molds for molding held by a gasket, a tape or the like. At this time, a degassing treatment, a filtration treatment or the like may be carried out, if necessary.

Subsequently, the monomers in the polymerizable composition that has been injected between the pair of molds for molding are polymerized, to cure the polymerizable composition between the molds for molding, thereby obtaining a cured product. Thereafter, the cured product is removed from the molds for molding, to obtain the cured product.

The polymerization of the monomers described above may be carried out by heating the polymerizable composition according to the present disclosure. The heating can be carried out, for example, using a heating apparatus including a mechanism for heating an object to be heated, in an oven, water or the like.

Polymerization conditions (such as polymerization temperature, polymerization time and the like) for polymerizing the monomers in the polymerizable composition according to the present disclosure can be set as appropriate, taking into consideration the composition of the polymerizable composition, the types and the used amounts of the monomers in the composition, the type and the used amount of the polymerization catalyst in the composition, the shape of the molds, and the like.

The polymerization temperature may be, for example, from -50°C to 150°C, from 10°C to 150°C, or the like.

The polymerization time may be, for example, from 1 hour to 200 hours, from 1 hour to 80 hours, or the like.

The cured product according to the present disclosure may be a cured product obtained by being subjected to a treatment such as annealing, after the polymerization of the monomers.

The annealing may be carried out, for example, at an annealing temperature of from 50°C to 150°C, from 90°C to 140°C, from 100°C to 130°C, or the like.

Since the cured product according to the present disclosure is a cured product of the polymerizable composition containing the episulfide compound, the cured product can have a high refractive index.

The cured product according to the present disclosure preferably has a refractive index (nd) of 1.71 or more, more preferably 1.72 or more, and still more preferably 1.73 or more.

Further, it is possible to obtain a cured product having any of various shapes, by changing the molds for molding that are used during the cast polymerization.

Therefore, the cured product according to the present disclosure can be suitably used, for example, as: a material for an optical material, such as an eyeglass lens, a camera lens or a light-emitting diode (LED); a material for a transparent resin member; or the like.

### [Optical Material]

The optical material (such as a lens; the same shall apply hereinafter) according to the present disclosure includes the cured product according to the present disclosure.

The optical material according to the present disclosure may be an optical material composed of the cured product according to the present disclosure, or may include the cured product according to the present disclosure and another element.

The other element may be, for example, another member, a coating layer provided on the cured product according to the present disclosure, or the like.

Since the optical material according to the present disclosure includes a cured product of the episulfide composition, the optical material can have a high refractive index.

The optical material according to the present disclosure preferably has a refractive index (nd) of 1.71 or more, more preferably 1.72 or more, and still more preferably 1.73 or more.

The optical material according to the present disclosure may be, for example, an eyeglass lens, a camera lens, a polarized lens, a light-emitting diode (LED) or the like.

### - Eyeglass Lens -

An eyeglass lens, as one example of the optical material according to the present disclosure, will be described below.

The eyeglass lens includes the cured product according to the present disclosure which has been molded in the form of a desired lens.

It is preferred that the eyeglass lens further includes a coating layer provided on one surface or on each of both surfaces of the cured product.

Specifically, the coating layer may be, for example, a primer layer, a hard coat layer, an anti-reflection layer, an anti-fog coating layer, an anti-fouling layer, a water-repellent layer or the like. These coating layers can be used singly, or alternatively, a plurality of the coating layers can be formed into a multilayer for use.

In the case of providing coating layers on both surfaces of the cured product, the same coating layer may be provided on each of the respective surfaces, or different coating layers may be provided on the respective surfaces.

The components of the coating layer can be selected as appropriate, depending on the purpose.

Examples of the components of the coating layer include resins (such as urethane resins, epoxy resins, polyester resins, melamine resins, and polyvinyl acetal resins), infrared absorbers, photostabilizers, antioxidants, photochromic compounds, dyes, pigments, and antistatic agents.

For example, the eyeglass lenses and the coating layers described in known literatures, such as JP-A No. 2002-194083, WO 2017/047745 and the like, can be referred to, as appropriate.

### EXAMPLES

Examples according to the present disclosure will be shown below. However, the present disclosure is not limited to the following Examples.

Hereinafter, the symbol "%" refers to "% by mass".

The terms "(SS)", "(RR)" and "(RS)" refer to a combination of S and S configurations, a combination of R and R configurations, and a combination of R and S configurations, respectively.

### [Production of Composition 1 Containing Episulfide Compound (A1-1) as Main Component]

In the present Production Example, a composition 1 containing an episulfide compound (A1-1) (hereinafter, also simply referred to as "(A1-1)") as a main component, was produced as a product of interest, using a racemic epichlorohydrin as a starting material.

The compound (A1-1) as used herein is a mixture of a compound having an RR configuration (hereinafter, referred to as "(A1-1RR)"), a compound having an SS configuration (hereinafter, referred to as "(A1-1SS)") and a compound having an RS configuration (hereinafter, referred to as "(A1-1RS)"), at the asymmetric carbon atoms.

The compound (A1-1RR) is one example of the optically active compound A, and more specifically, one example of a compound in which both of the asymmetric carbon atom C*¹ and the asymmetric carbon atom C*² in Formula (A1) have an R configuration.

The compound (A1-1SS) is one example of the optically active compound A, and more specifically, one example of a compound in which both of the asymmetric carbon atom C*¹ and the asymmetric carbon atom C*² in Formula (A1) have an S configuration.

The compound (A1-1RS) is one example of the optically active compound A, and more specifically, one example of a compound in which one of the asymmetric carbon atom C*¹ and the asymmetric carbon atom C*² in Formula (A1) has an R configuration, and the other one of the asymmetric carbon atoms therein has an S configuration.

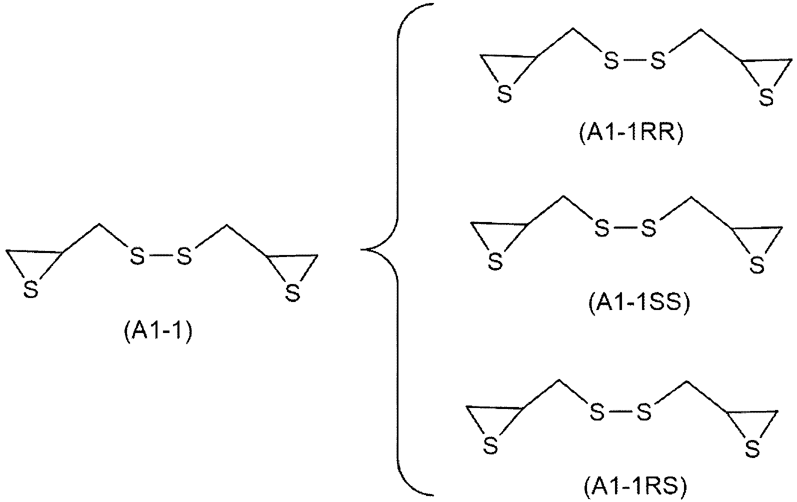

Production Example of the composition 1 containing the compound (A1-1) as a main component will be described below in detail.

### < Synthesis of Intermediate a1, which is Racemic 3-Chloro-1-mercapto-2-propanol >

A racemic epichlorohydrin as a starting material was allowed to react with H₂S to produce an intermediate a1, which is a racemic 3-chloro-1-mercapto-2-propanol (see the following reaction scheme).

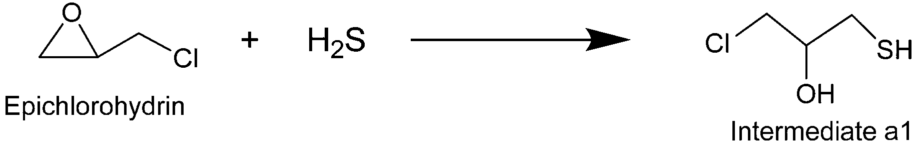

Detailed operation will be described below.

Into a reaction flask equipped with a stirring bar, a thermometer, a gas inlet pipe and a condenser, a racemic epichlorohydrin (462.4 g; 5.00 mol), methanol (800 ml), a 48% by mass aqueous solution of NaSH (6.0 g; the amount of NaSH: 2.9 g) were introduced, and the internal temperature of the flask was maintained at a temperature of from 0 to 5°C with stirring. Thereafter, hydrogen sulfide gas (277.5 g; 7.500 mol) was blown into the reaction system through the gas inlet pipe over 2 hours, and the resulting mixture was aged at 5°C for 3 hours to allow the epichlorohydrin to react with the hydrogen sulfide, thereby obtaining a reaction liquid. After removing methanol as the solvent from the thus obtained reaction liquid, the resulting concentrate was subjected to Smith's distillation, to obtain the intermediate a1 (namely, racemic 3-chloro-1-mercapto-2-propanol) having a purity of 99%, on the distillate side.

### < Synthesis of Intermediate b1, which is Racemic Halohydrin Compound >

The intermediate a1 obtained as described above was used as a starting material, to obtain an intermediate b1, which is a racemic halohydrin (see the following reaction scheme).

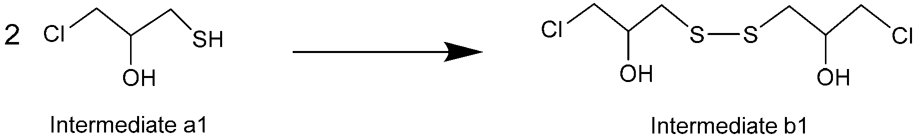

Detailed operation will be described below.

To the intermediate a1 (553.9 g; 4.330 mol) obtained as described above, pure water (445.1 g), sodium hydrogen carbonate (380.8 g) and methanol (123.6 g) were added. Thereafter, bromine (362.1 g; 2.266 mol) was added to the resulting mixture over 10 hours, while stirring the mixture under a nitrogen atmosphere with the internal temperature maintained at 10°C.

The stirring of the thus obtained liquid was continued for 1 hour while maintaining the internal temperature at 10°C, to allow the liquid to age, thereby obtaining a reaction liquid containing the intermediate b1.

### < Synthesis of Intermediate c1, which is Racemic Epoxy Compound >

The intermediate c1 obtained as described above was used as a starting material, to obtain an intermediate c1, which is a racemic epoxy compound (see the following reaction scheme).

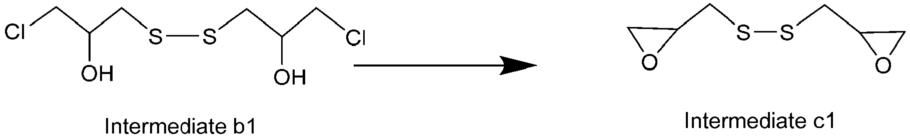

Detailed operation will be described below.

Toluene (93.4 g) was added to the reaction liquid containing the intermediate b1, which had been obtained as described above. To the resulting mixture, a 25% by mass aqueous solution of NaOH (824.2 g; 5.151 mol) was added dropwise over 3 hours, while stirring and maintaining the internal temperature at 10°C. Further, the stirring was continued for 3 hours while maintaining the internal temperature at 10°C, to allow the mixture to age.

Subsequently, pure water (203.3 g) was added to the aged mixture, followed by stirring for 15 minutes to separate the resulting liquid into an organic layer and an aqueous layer, and then the aqueous layer was discharged. A 13% by mass aqueous solution of NaCl (71.2 g) was added to the remaining organic layer, followed by stirring to separate the resulting liquid into an organic layer and an aqueous layer. Further, acetic acid was added thereto with stirring until the aqueous layer showed a pH of from 5 to 6. Thereafter, the aqueous layer was discharged.

The remaining organic layer was concentrated under reduced pressure, to obtain a crude product (365.3 g; purity: 97.6%) containing a racemic bis(2,3-epoxypropyl)disulfide (namely, the intermediate c1).

### < Synthesis of Intermediate d1, which is Racemic Thiouronium Salt >

The intermediate c1 obtained as described above was converted into a salt of thiouronium, to synthesize an intermediate d1, which is a racemic thiouronium salt.

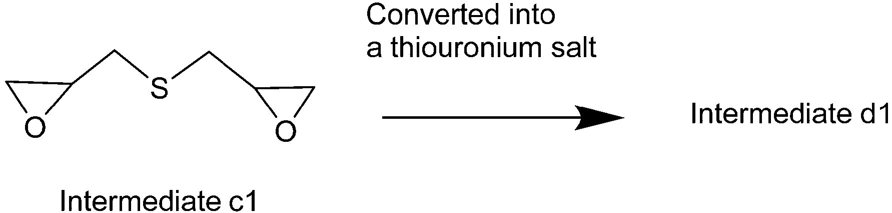

Detailed operation will be described below.

A quantity of 72.0 g (1.2 mol) of acetic acid, thiourea (313.0 g (4.111 mol), pure water (684.1 g), and methanol (533.3 g) were introduced into another flask. While maintaining the internal temperature of the resulting mixture as a base liquid ("shiki-eki" in Japanese) at 15°C, the above-described crude product (365.3 g; 2.000 mol in terms of the amount of the intermediate c1) containing the intermediate c1 was added dropwise thereto through a dropping funnel 1, and 88% by mass formic acid (157.0 g) was added dropwise thereto through a dropping funnel 2, respectively, over 5 hours.

After the completion of the dropwise addition of these liquids, the resulting mixture was further stirred for 3 hours while maintaining the internal temperature at 15°C, to obtain a reaction liquid containing the intermediate d1, which is a racemic thiouronium salt.

### < Production of Crude Product Containing Episulfide Compound (A1-1) >

The above-described reaction liquid containing the intermediate d1 was used to produce a crude product (hereinafter, also referred to as "crude (A1-1)") containing the episulfide compound (A1-1) (see the following reaction scheme).

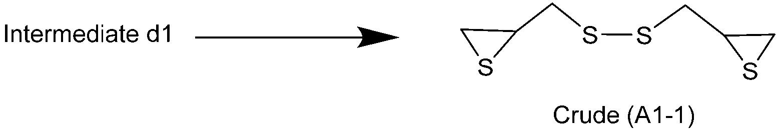

Detailed operation will be described below.

Methyl isobutyl ketone (MIBK) (760.7 g) was introduced all at once, into a flask containing the reaction liquid containing the intermediate d1. Subsequently, a 10% by mass ammonia water (679.7 g; 3.991 mol in terms of the amount of ammonia) was added dropwise to the flask over 2 hours. Further, the resulting mixture was allowed to age for 2 hours with stirring, while maintaining the internal temperature at 10°C. The thus obtained liquid was left to stand, subjected to liquid separation, and the aqueous layer was discarded.

The remaining organic layer was washed with 1056.7 g of a 13% by mass aqueous solution of NaCl and 6 g of a 10% by mass ammonia water, and then washed with a 13% by mass aqueous solution of NaCl (1056.7 g), acetic acid (11.9 g), and methanol (126.7 g).

The organic layer after the washing was concentrated under reduced pressure, to obtain the crude product (hereinafter, also referred to as "crude (A1-1)") (393.3 g) containing the episulfide compound (A1-1).

### < Purification (Production of Composition 1 Containing Episulfide Compound (A1-1) as Main Component) >

The crude (A1-1) described above was subjected to column purification, to obtain a composition 1 which contains the purified episulfide compound (A1-1) as a main component, and also contains trace amounts of impurities.

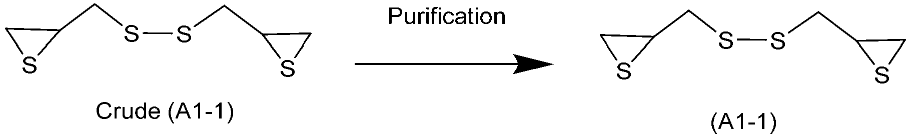

Detailed operation will be described below.

The crude (A1-1) described above was dissolved in a large amount of methylcyclohexane, and the components that had not been completely dissolved and remained partially were removed. The resulting solution of the compound (A1-1) was purified by passing the solution through a silica gel column. The silica gel column fraction thereof was concentrated, and subjected to a treatment to remove low boiling point components, to obtain the composition 1 which contains the purified episulfide compound (A1-1) as a main component, and also contains trace amounts of impurities.

### [Separation of Composition 1 Containing Compound (A1-1) as Main Component (Production of Crystal Composition and Liquid Composition)]

The composition 1 containing the compound (A1-1) as a main component, which had been obtained as described above, was separated into:
a crystal composition containing the compounds (A1-1RR) and (A1-1SS) as main components; and
a liquid composition containing the compound (A1-1RS) as a main component.

Detailed operation will be described below.

The composition 1 obtained as described above was placed into a transparent glass bottle, and stored in a refrigerator at 5°C for one week to allow crystals to form. The upper portion of the composition 1 after the storage was in the state of a clear liquid, and the lower portion thereof was in a state in which white crystals were precipitated. The composition 1 after the storage was separated into a supernatant which is the upper portion, and a crystal portion which is the lower portion, by decantation.

The crystal portion was introduced into ethyl acetate which had been cooled to a temperature of -25°C or lower, and sludged slowly. Then the stirring was stopped, and the resultant was placed in a freezer controlled to -25°C for 24 hours, as it is (the operation up to this point is hereinafter referred to as "ethyl acetate sludge". Subsequently, a slurry liquid after the ethyl acetate sludge was quickly filtered, and the crystal portion that had remained as a residue was collected. The thus collected crystal portion was subjected to the operations of the ethyl acetate sludge and the filtration, two more times.

The crystal portion which had been subjected to the operations of the ethyl acetate sludge and the filtration in a total of three times, as described above, was dried under high vacuum for 24 hours, using a desiccator, to obtain the crystal composition containing the compounds (A1-1RR) and (A1-1SS) as main components.

The ratio of optical isomers of the compound (A1-1) in the crystal composition was analyzed as will be described later.

Further, the supernatant was concentrated under reduced pressure, and further subjected to a treatment to remove low boiling point components, to obtain the liquid composition containing the compound (A1-1RS) as a main component.

The ratio of optical isomers of the compound (A1-1) in the liquid composition was analyzed as will be described later.

### [Analysis of Ratio of Optical Isomers in Crystal Composition and Liquid Composition]

The component analysis of the compound (A1-1) in the crystal composition and the compound (A1-1) in the liquid composition was carried out by high performance liquid chromatography (HPLC), under the following measurement conditions.

The results are shown in Table 1.

### - Measurement Conditions for HPLC -

| | |
|---|---|
| Columns | chiral separation columns AD-H; 0.46 cm diameter × 25 cm; two tandemly arranged columns; manufactured by Daicel Chemical Industries, Ltd. |
| Mobile phase | hexane: isopropyl alcohol (IPA) = 98:2 (volume ratio) |
| Flow rate | 0.7 ml/min |
| Oven temperature | 29°C |
| Measured wavelength | 215 nm |

**[Table 1]**

| | | Crystal composition | Liquid composition |
|---|---|---|---|
| Main component(s) in composition | | A1-1RR + A1-1SS | A1-1RS |
| Component analysis (% by area) | Peak area RS | 7.0 | 72.9 |
| | Peak area RR + Peak area SS | 93.0 | 27.1 |
| | Total peak area T (= Peak area RS + Peak area RR + Peak area SS) | 100 | 100 |

As shown in Table 1, the total peak area T (= the peak area RS + the peak area RR + the peak area SS), the peak area RR + the peak area SS, and the peak area RS, were determined for each of the compound (A1-1) in the crystal composition and the compound (A1-1) in the liquid composition.

Specifically, it has been confirmed that the main components in the crystal composition are the compounds (A1-1RR) and (A1-1SS), because a large value of the peak area RR + peak area SS was observed in the analysis results of the crystal composition.

In contrast, it has been confirmed that the main component in the liquid composition is the compound (A1-1RS), because a large value of the peak area RS was observed in the analysis results of the liquid composition.

### [Examples 1 to 3 and Comparative Example 1]

### < Production of Episulfide Compositions >

The crystal composition and the liquid composition described above were blended as appropriate, to obtain each of the episulfide compositions of Examples 1 to 3 and Comparative Example 1 shown in Table 2.

Conditions for analyzing the components in each episulfide composition are the same as the conditions for the component analysis carried out for each of the crystal composition and the liquid composition.

### < Production of Polymerizable Compositions >

Using each of the episulfide compositions of Examples 1 to 3 and Comparative Example 1, each corresponding polymerizable composition was produced in the following manner.

To a conical beaker, dicyclohexylmethylamine (15 mg) and dimethylcyclohexylamine (3 mg) as polymerization catalysts, and TINUVIN PS (manufactured by BASF Japan Ltd.) (0.165 g) as an ultraviolet absorber were introduced. The episulfide composition (15.000 g) of any one of Examples 1 to 3 and Comparative Example 1 was introduced into the beaker, followed by stirring and mixing. After confirming that the TINUVIN PS had dissolved by the stirring and mixing, a mixture (1.500 g) [polythiol composition] of 4,8-dimercaptomethyl-1,11-dimercapto-3,6,9-trithiaundecane, 4,7-dimercaptomethyl-1,11-dimercapto-3,6,9-trithiaundecane and 5,7-dimercaptomethyl-1,11-dimercapto-3,6,9-trithiaundecane was introduced, followed by mixing, to obtain a polymerizable composition.

### < Production of Cured Products (Resin Molded Articles) >

Each polymerizable composition obtained as described above was filtered with a TEFE filter having a retention particle size of 3 µm. The resulting filtrate (namely, the polymerizable composition) was subjected to degassing under reduced pressure at a temperature of from 15°C to 25°C for 1 hour. The polymerizable composition that had been subjected to the degassing under reduced pressure was injected between a pair of glass molds fixed with a tape. As the "pair of glass molds fixed with a tape", those for producing a 2 mm flat plate were used.

Subsequently, the pair of glass molds into which the polymerizable composition had been injected was placed in an oven.

Then the internal temperature of the oven was maintained, first, at 30°C for 10 hours, then increased from 30°C to 120°C over 8 hours, then maintained at 120°C for 2 hours, and then decreased from 120°C to 60°C over 1 hour. By the process as described above, monomers (namely, the episulfide compounds and the polythiol compound) in the polymerizable composition were polymerized, to form a cured product (namely, a resin molded article) of the polymerizable composition between the pair of glass molds.

Thereafter, the resin molded article was released from the pair of glass molds, to obtain a resin molded article in the form of a flat plate having a thickness of 2 mm, as the cured product of each polymerizable composition.

### < Evaluation >

### (Haze of Cured Product)

The haze of each cured product (namely, each resin molded article in the form of a flat plate having a thickness of 2 mm) obtained as described above was measured, using a NDH 2000 (manufactured by Nippon Denshoku Industries Co., Ltd.), and in accordance with "Test Method of Total Light Transmittance of Plastic Transparent Materials" defined in JIS K7361-1, "Test Methods of Optical Properties of Plastics" defined in JIS K7105, and "Plastics - Method of Determining Haze of Transparent Materials" defined in JIS K7136.

The haze of each cured product was evaluated based on the thus determined haze, in accordance with the following evaluation criteria.

The results are shown in Table 2.

In the following evaluation criteria, those in which the haze of the cured product is evaluated as most reduced, are classified as Rank A.

### - Evaluation Criteria for Haze of Cured Product -

A: The haze is less than 3.
B: The haze is 3 or more but less than 20.
C: The haze is 20 or more.

### (Maximum point stress of bending of Cured Product)

In each of Examples 1 to 3, the maximum point stress of bending of the cured product was also evaluated, in addition to the evaluation of the haze of the cured product described above.

A test piece having a length of 65 mm and a width of 25 mm was cut out from each cured product (namely, each resin molded article in the form of a flat plate having a thickness of 2 mm) obtained as described above, and a cross section of the thus cut-out test piece was polished with a No. 800 file, to obtain a flat plate-like test piece having a length of 65 mm, a width of 25 mm, and a thickness of 2 mm.

The thus obtained flat plate-like test piece was subjected to a three-point bending test, using an autograph (manufactured by Shimadzu Seisakusho Co. Ltd.), with the width between lower pedestals to be placed during the measurement of the flat plate-like test piece set to 34 mm.

The maximum point stress in the above-described three-point bending test was determined, and the maximum point stress of bending was evaluated in accordance with the following evaluation criteria.

The results are shown in Table 2.

In the following evaluation criteria, those in which the maximum point stress of bending of the cured product is evaluated as the best, are classified as Rank AA.

### - Evaluation Criteria for Maximum point stress of bending of Cured Product -

AA: The maximum point stress is 125 N/mm² or more.
A: The maximum point stress is 100 N/mm² or more but less than 125 N/mm².
B: The maximum point stress is 90 N/mm² or more but less than 100 N/mm².
C: The maximum point stress is less than 90 N/mm².

**[Table 2]**

| | | Comparative Example 1 | Example 1 | Example 2 | Example 3 |
|---|---|---|---|---|---|
| Component analysis of episulfide composition (% by area) | Peak area ratio [RS/T] | 72.9 | 61.6 | 47.3 | 14.0 |
| | Peak area RS | 72.9 | 61.6 | 47.3 | 14.0 |
| | Peak area RR + Peak area SS | 27.1 | 38.4 | 52.7 | 86.0 |
| | Total peak area T (= Peak area RS + Peak area RR + Peak area SS) | 100 | 100 | 100 | 100 |
| Evaluation results | Haze of cured product | C | A | A | A |
| | Maximum point stress of bending of cured product | - | AA | B | B |

As shown in Table 2, the haze of the cured product was reduced in each of Examples 1 to 3 in which an episulfide composition having a peak area ratio [RS/T]) of from 10% by area to 70% by area had been used.

In contrast, it was not possible to reduce the haze of the cured product in Comparative Example 1 in which an episulfide composition having a peak area ratio [RS/T] of more than 70% by area had been used.

Among Examples 1 to 3, Example 1 in which an episulfide composition having a peak area ratio [RS/T] of more than 50% by area and less than or equal to 70% by area had been used, showed a better maximum point stress of bending of the cured product, as compared to other Examples.

The disclosure of Japanese Patent Application No. 2023-009655 filed on January 25, 2023 is incorporated herein by reference in their entirety.

All publications, patent applications, and technical standards mentioned in the present specification are incorporated herein by reference to the same extent as if each individual publication, patent application, or technical standard was specifically and individually indicated to be incorporated by reference.

## Claims

1. An episulfide composition comprising an optically active compound A, wherein:
the optically active compound A comprises one disulfide bond and two episulfide rings, and
one of the two episulfide rings is an episulfide ring 1 containing an asymmetric carbon atom C*¹, and another of the two episulfide rings is an episulfide ring 2 containing an asymmetric carbon atom C*²,
wherein the optically active compound A comprises:
at least one of an optically active compound A-SS in which both of the asymmetric carbon atom C*¹ and the asymmetric carbon atom C*² have an S configuration, or an optically active compound A-RR in which both of the asymmetric carbon atom C*¹ and the asymmetric carbon atom C*² have an R configuration; and
an optically active compound A-RS in which one of the asymmetric carbon atom C*¹ and the asymmetric carbon atom C*² has an R configuration, and another of the asymmetric carbon atoms has an S configuration; and
wherein a ratio of a peak area RS of the optically active compound A-RS to a total peak area T of the optically active compound A-SS, the optically active compound A-RR and the optically active compound A-RS, as measured by high performance liquid chromatography, is from 10% by area to 70% by area.

2. The episulfide composition according to claim 1, wherein the ratio of the peak area RS to the total peak area T is more than 50% by area and less than or equal to 70% by area.

3. The episulfide composition according to claim 1, wherein the ratio of the peak area RS to the total peak area T is from 10% by area to 50% by area.

4. The episulfide composition according to any one of claims 1 to 3, wherein the optically active compound A comprises a compound represented by the following Formula (A1): 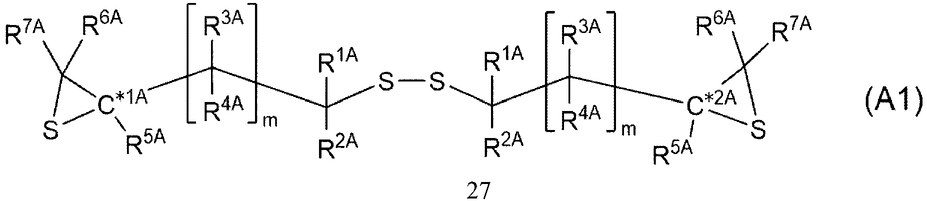 wherein, in Formula (A1), each of R^{1A} to R^{7A} independently represents a hydrogen atom or an optionally substituted monovalent hydrocarbon group; m represents an integer from 0 to 3; C*^{1A} represents the asymmetric carbon atom C*¹ in the compound represented by Formula (A1), and C*^{2A} represents the asymmetric carbon atom C*² in the compound represented by Formula (A1); and each of R^{1A} to R^{7A} and m, which are present in pluralities, may be the same as, or different from, each other.

5. The episulfide composition according to any one of claims 1 to 3, wherein a content of the optically active compound A is 90% by mass or more with respect to a total amount of the episulfide composition.

6. A polymerizable composition comprising the episulfide composition according to any one of claims 1 to 3, and a polymerization catalyst.

7. The polymerizable composition according to claim 6, wherein the polymerizable composition is used in production of an optical material.

8. A cured product of the polymerizable composition according to claim 6.

9. An optical material comprising a cured product of the polymerizable composition according to claim 6.

10. A lens comprising a cured product of the polymerizable composition according to claim 6.
